# EUROPÄISCHE PATENTANMELDUNG

(11) **EP 2 942 388 A1**
(43) Veröffentlichungstag der Anmeldung: **11.11.2015**
(21) Anmeldenummer: 14167591.8
(22) Anmeldetag: 08.05.2014
(51) Int. Cl.: C12M 1/107, C12M 1/00

(54) **Verfahren zum Erzeugen von Biogas und Biogasanlage**

(71) Anmelder: M&M engineering GmbH, 39100 Bozen (IT)
(72) Erfinder: Mintah, Gunnar, 39100 Bozen (IT)
(74) Vertreter: Krauss, Jan

(57) **Zusammenfassung**

Bei einem Verfahren zum Erzeugen von Biogas aus organischer Biomasse, wie Energiepflanzen, Gülle, Festmist oder Bioabfällen, in einer Biogasanlage mit wenigstens einem Fermentationsbehälter und einem Hydrolysereaktor wird Biomasse unter Ausbildung eines Gärrests in dem wenigstens einen Fermentationsbehälter vorvergoren; im Anschluss an das Vorvergären zumindest ein Anteil des Gärrests in dem Hydrolysereaktor chemisch hydrolysiert; und der hydrolysierte Gärrest nachvergoren.

## Beschreibung

Die Erfindung betrifft ein Verfahren und eine Biogasanlage zum Erzeugen von Biogas aus Biomasse, wie Energiepflanzen, Gülle, Festmist, Bioabfällen oder landwirtschaftlichen Nebenprodukten, z.B. Schlachtabfällen.

In einer Biogasanlage wird durch Vergärung eines Anlagensubstrats methanhaltiges Gas erzeugt, das zur Strom- und/oder Wärmeerzeugung verwendet werden kann. Das Analgensubstrat einer Biogasanlage besteht größtenteils, d.h. zu wenigstens 80% aus organischer, mikrobiell abbaubarer Biomasse. Der Gärvorgang läuft dabei in einem luftdicht abgeschlossenen Fermentationsbehälter oder Gärreaktor unter anaeroben Bedingungen ab. Mikroorganismen verstoffwechseln dabei in der Biomasse enthaltene Kohlehydrate, Fette und Proteine und produzieren Methan und Kohlendioxid.

Viele Bestandteile des Anlagensubstrats weisen allerding eine komplexe chemische Struktur auf, die sich durch die Mikroorganismen nur langsam oder gar nicht verstoffwechseln lässt, was zu einer nicht zufriedenstellenden Gasausbeute bei der Erzeugung von Biogas führt.

Aus der Druckschrift WO2006/042551A1 sind ein Verfahren und eine Anlage zum Erzeugen von Biogas aus organischem Abfall bekannt, bei denen organischer Abfall vergoren wird, der vergorene organische Abfall einer anaeroben Hydrolyse unterzogen und anschließend der hydrolysierte organische Abfall weiter vergoren wird. Durch Hydrolysieren der organischen Abfälle im Anschluss an eine Vorvergärung soll eine Verbesserung der Biogasausbeute erreicht sein.

Allerdings zeigte sich, dass für die Zerlegung der komplexen Bestandteile der organischen Biomasse der organische Abfall bei dem bekannten Verfahren im Hydrolyseschritt auf sehr hohe Temperaturen erhitzt und über lange Zeit in diesem Hochtemperaturbereich gehalten werden muss. Die höhere Gasausbeute wird somit durch einen höheren Betriebsenergieaufwand verkauft, so dass bei einer Gesamtenergiebetrachtung sich lediglich eine geringfüge Verbesserung gegenüber der Ursprungssituation ergibt. Zudem erfordern die langen Verweilzeiten große Hydrolysebehälter.

Es ist Aufgabe der Erfindung, die Nachteile des Stands der Technik zu überwinden und insbesondere ein Verfahren und eine Biogasanlage zum Erzeugen von Biogas aus organischer Biomasse bereitzustellen, die bei geringem Raumbedarf ein verbessertes Verhältnis von Gasausbeute zu Betriebskosten erreichen.

Diese Aufgabe wird durch das Verfahren und den Gegenstand der unabhängigen Ansprüche 1 und 9 gelöst.

Danach ist ein Verfahren zum Erzeugen von Biogas aus organischer Biomasse, wie Energiepflanzen, Gülle, Festmist oder Bioabfällen, in einer Biogasanlage mit wenigstens einem Fermentationsbehälter und einem Hydrolysereaktor geschlagen, bei dem die Biomasse unter Ausbildung eines Gärrest in dem wenigstens einen Fermentationsbehälter vorvergoren wird. Die Biomasse wird als Hauptbestandteil, d. h. mit einem Anteil von wenigstens 75%, eines Anlagensubstrats in den wenigstens einen Fermentationsbehälter zugeführt. Insbesondere werden das Anlagensubstrat und die in dem Anlagensubstrat enthaltene Biomasse auf eine Vorvergärungstemperatur erwärmt. Bei dem Vorvergärungsprozess kann entstehendes Biogas aus dem wenigstens einen Fermentationsbehälter entfernt und gespeichert werden. Nach Abschluss der Vorvergärung wird der Gärrest aus dem Fermentationsbehälter in den Hydrolysereaktor abgezogen. Im Anschluss an das Vorvergären wird zumindest ein Anteil des Gärrests in dem Hydrolysereaktor hydrolysiert. Im Anschluss an die Hydrolyse wird der hydrolysierte Gärrest in einem Fermentationsbehälter nachvergoren.

Erfindungsgemäß wird der vorvergorene Gärrest chemisch hydrolysiert, d. h. dass die Aufspaltung der chemischen Verbindungen in der Biomasse durch Zugabe einer vorbestimmten Menge den PH-Wert des vorvergorenen Gärrests veränderten Chemikalie dem Hydrolysereaktor zugegeben wird. Es zeigte sich, dass durch die Veränderung des PH-Werts, insbesondere durch Erhöhung der H+ bzw. OH- Konzentration im Hydrolysereaktor die Aufspaltung der Zucker, Stärke und Proteinanteile der Biomasse erheblich beschleunigt werden konnte. Da die aus der Spaltung resultierenden Monomere durch die Mikroorganismen der anschließenden Nachvergärung vergleichsweise effizient abgebaut werden, konnte eine deutlich erhöhte Gasausbeute beim Nachvergären des hydrolysierten Gärrests erreicht werden.

Bei einer Weiterbildung wird dem Gärsubstrat für die chemische Hydrolyse eine Chemikalie hinzugegeben, um eine Gärrestchemikalienmischung zu bilden. Vorzugsweise enthält die Chemikalie eine Säure oder Base. Besonders bevorzugt weißt die Chemikalie einen PH-Wert von wenigstens 12, vorzugsweise wenigstens etwa 13 auf. Bei einer alternativen Ausgestaltung weist die Chemikalie einen PH-Wert von höchstens 3, vorzugsweise höchstens etwa 2 auf. Insbesondere wird die Gärrestchemikalienmischung während der Hydrolyse durch ein Rührwerk durchmischt gehalten.

Bei einer bevorzugten Weiterbildung des Verfahrens wird während der Gesamtdauer der chemischen Hydrolyse, oder zumindest über einen überwiegenden Zeitraum, wie wenigstens 75% der Gesamtdauer, der chemischen Hydrolyse der PH-Wert der Gärrestchemikalienmischung zwischen etwa 9 und etwa 13, vorzugsweise zwischen etwa 10 und etwa 12 eingestellt. Es zeigte sich, dass die beim Hydrolyseprozess freigesetzten organischen Säuren, sofern die chemische Hydrolyse zumindest bei Einleitung der Hydrolyse im alkalischen Bereich eingestellt wird, das hydrolysierte Gärsubtrat fast vollständig neutralisieren können. Da für die Nachvergärung neutrales Niveau wünschenswert ist, können zusätzliche Verfahrensschritte zur Aufbereitung des hydrolysierten Gärrests für die Nachvergärung vorzugsweise vollständig entfallen.

Bei einer Weiterbildung wird der vorvergorene Gärrest aerob, vorzugsweise schwach aerob hydrolysiert. Überraschenderweise konnte mit einer insbesondere schwach aeroben Führung der Hydrolyse, ein mikrobiologisch äußerst effizient verarbeitbares hydrolysiertes Gärsubstrat bei kürzerer Hydrolysedauer im Vergleich zu einer anaeroben Hydrolyse erreicht werden.

Bei einer bevorzugten Weiterbildung wird der vorvergorene Gärrest auf eine vorbestimmte Hydrolysetemperatur erwärmt bevor und/oder während er hydrolysiert wird, insbesondere jedoch nach der Vorvergärung. Insbesondere wird der vorvergorene Gärrest in einem Wärmetauscher erwärmt. Vorzugsweise wird der Gärrest durch zwei in Reihe geschaltete Wärmetauscher geleitet. Insbesondere arbeitet der in Reihe zuerst geschaltete Wärmetauscher bidirektional. Insbesondere beträgt die Hydrolysetemperatur zwischen 20°C und 100°C, vorzugsweise zwischen 60°C und 90°C, besonders bevorzugt zwischen etwa 75°C und 85°C.

Einer Weiterbildung wird der hydrolysierte Gärrest vor dem Nachgären von einer Hydrolysetemperatur auf eine vorbestimmte Nachvergärungstemperatur abgekühlt. Insbesondere wird der hydrolysierte Gärrest dazu durch den ersten insbesondere bidirektional arbeitenden Wärmetauscher geleitet. Insbesondere wird die Nachvergärungstemperatur des Gärrests auf zwischen 20°C und etwa 70°C eingestellt, vorzugsweise auf zwischen etwa 50°C und etwa 60°C.

Es zeigte sich überraschenderweise, dass die Kombination von sauer oder alkalisch geführter Hydrolyse und Zuführung von Wärmeenergie während der Hydrolyse eine deutlich überproportionale Beschleunigung des Hydrolyseprozesses bewirkte, als dies in Anbetracht der Ergebnisse bei Zufuhr von Wärmeenergie in bekannten Hydrolyseprozessen zu erwarten gewesen wäre.

Bei einer bevorzugten Weiterbildung der Erfindung werden die bei der Hydrolyse im Hydrolysereaktor entstehenden Gase aus dem Hydrolysereaktor entfernt. Insbesondere werden die entfernten Gase unter Verwendung eines Gaswäschers mit Schwefelsäure angereichert. Es zeigte sich, dass das Entfernen der im Hydrolyseprozess entstehenden Gase einen Stickstoff und Schwefelabbau im hydrolysierten Gärrest auslöste. Dadurch wird eine verbesserte Nachvergärung erreicht und die Biogasqualität im Hinblick auf die Verbrennung in Gasmotoren verbessert. Zudem konnte eine Unbedenklichkeitsschwelle bezüglich der landwirtschaftlichen Weiterverarbeitung des Gärrests unterschritten werden. Beim Anreichern der entfernten Gase, insbesondere des in dem entfernten Gase enthaltenen Ammoniaks mit Schwefelsäure im Gaswäscher, wird Ammoniumsulfat erzeugt, dass als Zusatzstoff für Düngemittel weiterverwertbar ist.

Bei einer Weiterbildung wird der hydrolysierte Gärrest vor dem Nachvergären mit einer den PH-Wert verändernden Chemikalie behandelt. Insbesondere wird der PH-Wert des Gärrests im Wesentlichen neutral eingestellt. Dieser Verfahrensschritt wird nur bedarfsweise ausgeführt, sofern die Hydrolyse im sauren Bereich geführt wird oder die bei der alkalischen Hydrolyse immanente Selbstneutralisierung nicht ausreicht.

Bei einer Weiterbildung wird der hydrolysierte Gärrest in demselben Fermentationsbehälter nachvergoren, in dem die Biomasse vorvergoren worden ist. Bei dieser Verfahrensführung ist besonders vorteilhaft, dass lediglich ein Fermentationsbehälter vorgesehen werden muss, wodurch sich der Verfahrensaufwand deutlich reduziert.

Insbesondere arbeitet das Verfahren gemäß der Funktionsweise der nachfolgend beschriebenen erfindungsgemäßen Biogasanlage.

Die Erfindung betrifft auch eine Biogasanlage zur Erzeugung von Biogas aus organischer Biomasse, wie Energiepflanzen, Gülle, Festmist oder Bioabfällen. Die Biogasanlage umfasst einen Fermentationsbehälter, in dem zu vergärende Biomasse oder ein zu vergärender Gärrest einbringbar ist und aus dem Biogas sowie ein vergorener Gärrest abziehbar sind, sowie einen Hydrolysereaktor, der mit dem Fermentationsbehälter verbindbar ist, um den vergorenen Gärrest aus dem Fermentationsbehälter aufzunehmen. Erfindungsgemäß ist wenigstens eine Dosiereinrichtung vorgesehen, die mit einem Chemikalienspeicher und dem Hydrolysereaktor verbunden und dazu ausgelegt ist, dem Hydrolysereaktor eine definierte Menge einer Chemikalie zuzuführen.

Bei einer Weiterbildung weist die Biogasanlage wenigstens einen Wärmetauscher auf, der mit dem Fermentationsbehälter und dem Hydrolysereaktor verbindbar und dazu ausgelegt ist, einen aus dem Fermentationsbehälter abgezogenen Gärrest zu erwärmen und/oder einen hydrolysierten Gärrest aus dem Hydrolysereaktor abzukühlen. Insbesondere ist der Wärmetauscher über ein Warmwasserleitungssystem mit einem Blockheizkraftwerk verbunden. Insbesondere weist die Biogasanlage ein Gasleistungsnetz zum Versorgen des Blockheizkraftwerks mit Biogas auf.

Die Erfindung betrifft insofern auch ein System zur Erzeugung von Elektro- und/oder Wärmeenergie, das eine Biogasanlage und ein mit der Biogasanlage über ein Gasleitungsnetz verbundenes Blockheizkraftwerk umfasst.

Bei einer bevorzugten Weiterbildung ist der Hydrolysereaktor chemikalienbeständig, insbesondere vollständig säure- und/oder basenbeständig ausgelegt. Insbesondere ist der Hydrolysereaktor basenbeständig bis zu einem PH-Wert von 13 ausgelegt.

Insbesondere weißt der Hydrolysereaktor eine Belüftungsvorrichtung zum Zuführen von Sauerstoff in dem Hydrolysereaktor auf, die insbesondere eine Luftdrosseleinrichtung zum Einstellen der zugeführten Sauerstoffmenge umfasst. Mittels einer genauen Dossierung der dem Hydrolysereaktor zugeführten Sauerstoffmenge lässt sich die Hydrolyse in einer schwachaeroben Verfahrensführung betreiben, die überraschenderweise eine Verkürzung der Hydrolysedauer bei gleichem Aufspaltungsergebnis erlaubte.

Bei einer bevorzugten Weiterbildung weist die Biogasanlage einen zweiten Fermentationsbehälter auf, der dem Hydrolysereaktor nachgeschaltet ist. Der zweite Fermentationsbehälter empfängt den hydrolysierten Gärrest des Hydrolysereaktors zur Nachvergärung.

Bei einer alternativen Weiterbildung umfasst die Biogasanlage nur einen Fermentationsbehälter, der dem Hydrolysereaktor sowohl vorgeschaltet als auch nachgeschaltet ist. Bei dieser Ausführung wird der einzige Fermentationsbehälter zur Vorvergärung und zur Nachvergärung genutzt, was den Wartungs- und Investitionsaufwand sowie den Platzbedarf minimiert.

Weitere Eigenschaften, Vorteile und Merkmale der Erfindung werden durch die folgende Beschreibung, bevorzugter Ausführung anhand der beiliegenden Zeichnungen deutlich, die zeigen:
- Figur 1:: Schematischer Aufbau einer erfindungsgemäßen Biogasanlage zur Ausführung des erfindungsgemäßen Verfahrens; und
- Figur 2:: Schematischer Aufbau einer alternativen Ausführung einer erfindungsgemäßen Biogasanlage zur Ausführung des erfindungsgemäßen Verfahrens.

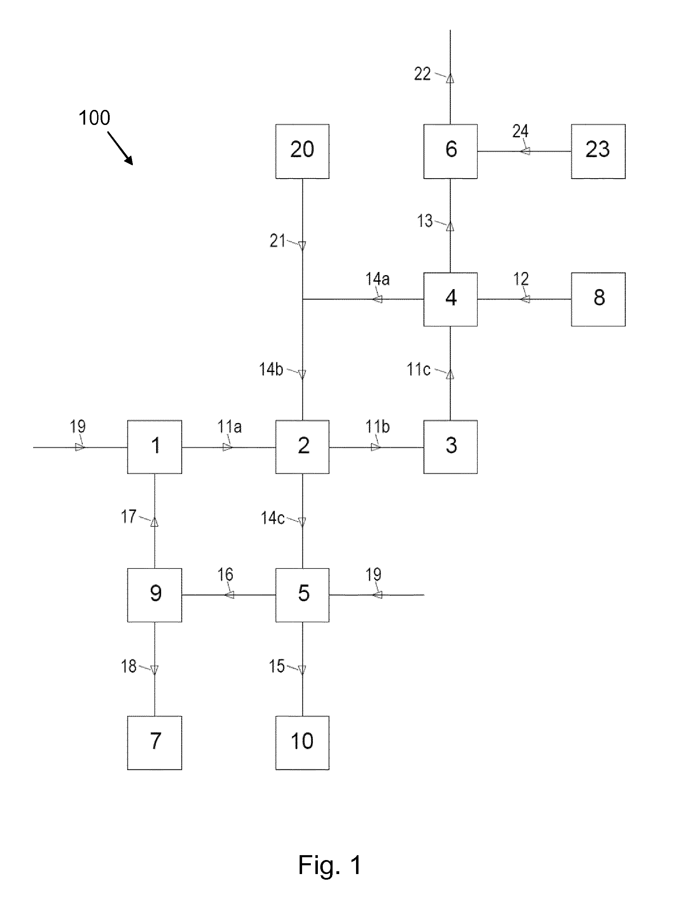

In Figur 1 ist eine erfindungsgemäße Biogasanlage (100) zum Erzeugen von Biogas aus organischer Biomasse dargestellt, die zwei Fermentationsbehälter (1, 5) aufweist. An die beiden Gärbehälter (1, 5) ist jeweils eine Biomasse Zuführleitung (19) angeschlossen. Die Fermentationsbehälter (1, 5) sind luftdicht abschließbar, um einen anaeroben Fermentationsprozess innerhalb der Behälter zu gewährleisten. Auch eine jeweilige Anschlussstelle der Zuführleitungen (19) an die Fermentationsbehälter (1, 5) sowie alle anderen nachfolgend genannten Anschlüsse an die Fermentationsbehälter (1, 5) können ebenfalls luftdicht ausgeführt sein, dass die anaerobe Fermentation nicht durch Luftzustrom beeinträchtigt wird.

Der Fermentationsreaktor (1) ist über eine Leitung (11a) mit einem Wärmetauscher (2) verbunden. Der Wärmetauscher (2) ist dazu ausgelegt, in einem ersten Betriebsmodus den durch den Wärmetauscher geführten Gärrest zu erwärmen und in einem zweiten Betriebsmodus den durch den Wärmtauscher (2) geführten Gärrest abzukühlen. Der Wärmetauscher (2) ist über eine Leitung (14c) mit dem zweiten Fermentationsbehälter (5) sowie über eine Leitung (11b) mit einem zweiten Wärmetauscher (3). Die Verbindungsleitung (11b) zwischen den Wärmetauschern kann wärmeisoliert sein, um Energieverluste beim stufenweisen Erwärmen und Weiterleiten des Gärrests zu vermeiden.

Eine Leitung (11c) verbindet den zweiten Wärmetauscher (3) mit einem Hydrolysereaktor (4). An dem Hydrolysereaktor (4) ist eine Dosiereinrichtung (8) für Chemikalien angeschlossen, die mit dem Hydrolysereaktor (4) über eine chemikalienbeständige Leitung (12) verbunden ist. Die Dosiereinrichtung (8) ist mit einem nicht näher dargestellten Chemikalienspeicher verbunden, der auch Bestandteil der Dosiereinrichtung sein kann und in dem ein von der Dosiereinrichtung an den Hydrolysereaktor (4) abzugebender Vorrat einer Chemikalie lagert.

Der Hydrolysereaktor (4) umfasst einen Aufnahmeraum, in dem der vorvergorene Gärrest mit einer von der Dosiereinrichtung (8) abgegebenen Chemikalie zusammengebracht vorgehalten werden. Eine Heizeinrichtung (nicht näher dargestellt) ist vorzugsweise in einer Wandung des Aufnahmeraums vorgesehen, mittels der eine Gärrestchemikalienmischung auf einer vorbestimmten Hydrolysetemperatur gehalten werden kann. Der Hydrolysereaktor (4) weist weiterhin ein in dem Aufnahmeraum angeordnetes Rührwerk (nicht näher dargestellt) auf, mittels dem die Gärrestchemikalienmischung insbesondere während der gesamten Verweildauer im Hydrolysereaktor (4) in Bewegung gehalten und/oder durchmischt wird.

In dem Hydrolysereaktor (4) ist eine Belüftungsvorrichtung (nicht näher dargestellt) eingebaut, die beispielsweise die Atmosphäre mit dem Aufnahmeraum verbindet und mittels der dem Hydrolysereaktor Sauerstoff geregelt zugeführt werden kann. Die Belüftungsvorrichtung weist eine Drosseleinrichtung auf (nicht näher dargestellt), die beispielsweise über einen Regelalgorithmus derart angesteuert wird, dass innerhalb des Hydrolysereaktors (4) ein schwach aerobes Milieu erreicht ist. Beispielsweise kann die Belüftungsvorrichtung durch eine oder mehrere Öffnungen in der Wandung des Hydrolysereaktors (4) realisiert sein, die bedarfsweise fluiddicht verschließbar ist/sind.

Der Hydrolysereaktor ist aus einem chemikalienbeständigen Material gefertigt oder zumindest an dessen mit der Gärrestchemikalienmischung in Kontakt kommenden Innenwandung chemikalienbeständig verkleidet oder beschichtet. Schließlich ist der Hydrolysereaktor an einen Gaswäscher (6) über die Leitung (13) angeschlossen.

Der Gaswäscher (6) kann eine nicht näher dargestellte Gastransportvorrichtung aufweisen, mittels der über die Leitung (13) bei der Hydrolyse entstehende Hydrolysegase aus dem Kopfbereich des Hydrolysereaktors (4) in den Gaswäscher (6) ausgeleitet werden. Die Hydrolysegase enthalten hohe Anteile an Kohlendioxid, Schwefelwasserstoff und Ammoniak. Im Gaswäscher (6) lässt sich der Ammoniakanteil der Hydrolysegase zu Ammoniumsulfat aufbereiten, das als Düngemittelzusatz verwertbar ist. Dazu ist der Gaswäscher (6) mit einer Schwefelsäurequelle (23) über eine Leitung (24) verbunden und insbesondere mit einer Regelung ausgestattet, die den Anteil der Hydrolysegase und der Schwefelsäure im Gaswäscher derart einstellt, das Ammoniumsulfat gewonnen wird. Der Gaswäscher (6) weißt einen Wertstoffauslass (22) auf, über den das gewonnene Ammoniumsulfat dem Gaswäscher (6) entnommen wird. Über die Ablassleitung (14a, 14b) ist der Hydrolysereaktor direkt mit dem Wärmetauscher (2) verbunden. In die Ablassleitung (14a, 14b) mündet außerdem eine Zuführleitung (21), die mit einem Neutralisationsdosierer (20) verbunden ist. Der Neutralisationsdosierer bezieht eine Chemikalie zur Neutralisierung der von der Dosiereinrichtung (8) zugeführten Chemikalie aus einem nicht näher dargestellten Chemikalienspeicher.

In einer nicht näher dargestellten Ausführung umfasst die Biogasanlage einen zentralen Chemikalienspeicher, der die von der Dosiereinrichtung (8) und die von dem Neutralisationsdosierer (20) verwendete Chemikalien sowie die von dem Gaswäscher benötigte Schwefelsäure bevorratet. Dazu weist der Chemikalienspeicher separate Abteile für die jeweilige Chemikalie auf. Insbesondere sind die Dosiereinrichtung (8) und der Neutralisationsdosierer (20) durch ein einziges Dosiermoduls mit einer gemeinsamen Steuerungslogik gebildet.

Der dem Wärmetauscher (2) nachgeschaltete Fermentationsbehälter (5) ist über eine Ablassleitung (16) mit einer Separiervorrichtung (9) verbunden. Die Separiervorrichtung (9) ist dazu ausgelegt, einen Feststoffanteil des aus dem Fermentationsbehälter (5) abgezogenen Gärrests von einem Flüssiganteil zu trennen. Die Separiervorrichtung (9) weißt einen Feststoffauslass auf, der über einen Feststoffleitung (18) mit einem Feststofflager (7) verbunden ist. Der von dem Gärrest abgespaltete Flüssiganteil, auch Rezirkulat genannt, wird aus einen Rezirkulatauslass der Separiervorrichtung (9) über eine für den Flüssigkeitstransport ausgelegte Rezirkulatleitung (17) dem ersten Fermentationsbehälter (1) bedarfsgemäß dossiert zugeführt.

Mit der Biogaseinlage (100) gemäß der oben beschriebenen Ausführung lässt sich das erfindungsgemäße Verfahren beispielsweise wie nachfolgend beschrieben realisieren:
Über die Zuführleitung (19) wird zunächst frische Biomasse in den Fermentationsbehälter (1) geleitet. Zur Optimierung des mikroorganischen Abbauprozesses kann Rezirkulat, das aus vorangehenden Anlagenläufen vorhanden ist, dem ersten Fermentationsbehälter (1) zugesetzt werden. Der Fermentationsbehälter (1) wird luftdicht abgeschlossen, so dass ein anaerober mikrobieller Gärprozess beginnt. Durch Vorerwärmung des Rezirkulats und/oder Erwärmung der Biomasse in dem ersten Fermentationsbehälter (1) wird die Temperatur des Biomasse enthaltenden Anlagensubstrats im Fermentationsbehälter auf etwa 40°C eingestellt. Die Rezirkulatleitung (17) und/oder der Fermentationsbehälter (1) können eine Heizeinrichtung aufweisen, mittels der die Temperatur möglichst konstant bei etwa 40°C gehalten wird. Dazu können auch ein Temperatursensor und eine Regelungselektronik vorgesehen sein. Der Vorvergärprozess wird über 10 bis 50 Tage, vorzugsweise über 20 bis 30 Tage aufrechterhalten. Durch den Vorvergärvorgang bildet sich aus dem ursprünglich frische Biomasse enthaltenden Anlagensubstrat ein Gärrest sowie von den Mikroorganismen produziertes Gas. Das Gas wird während des Vorvergärvorgangs aus dem Fermentationsbehälter (1) abgezogen.

Nach der Vorvergärung gelangt der Gärrest über die Leitung (11a) in den ersten Wärmetauscher (2), in dem der Gärrest von etwa 40°C auf etwa 65°C erwärmt wird. Aus dem ersten Wärmetauscher (2) wird der Gärrest für die Leitung (11b) dem zweiten Wärmetauscher (3) zugeleitet, um eine weitere Erwärmung des Gärsubstrats auf etwa 80°C zu realisieren. Die Wärmetauscher (2, 3) sind über ein nicht näher dargestelltes Warmwasserleitungsnetz mit einer Wärmequelle verbunden. Vorzugsweise wird als Wärmequelle ein Blockheizkraftwerk (BHKW) eingesetzt, das mit dem von der Anlage erzeugten Biogas betrieben werden kann.

Aus dem Wärmetauscher (3) gelangt das auf 80°C erwärmte Gärsubstrat in den Hydrolysereaktor (4). Mittels der Dosiereinrichtung (8) wird dem Hydrolysereaktor (4) und dem darin aufgenommenen Gärrest über die Leitung (12) Lauge, wie Natriumhydroxid (NaOH) zugeleitet, um eine Gärrestchemikalienmischung zu bilden. Das Rührwerk des Hydrolysereaktors (4) durchmischt die Gärrestchemikalienmischung fortwährend.

In dem Hydrolysereaktor (4) wird der Gärrest dann chemisch und thermisch hydrolysiert. Dabei werden in dem Gärrest enthaltene von den Mikroorganismen schwer abbaubare polymer Stärken, Cellulosen und Mehrfach-Zucker in Monomere zerlegt. Die Dosiereinrichtung (8) kann eine Sensorik sowie eine Regelung aufweisen mittels der der PH-Wert der Gärrestnatronlaugenmischung auf einen PH-Wert zwischen 10 und 12 eingestellt wird. Eine Regelung der Dosiereinrichtung (8) kann auch derart ausgelegt sein, dass sich der PH-Wert der Gärrestchemikalienmischung über die Verweildauer im Hydrolysereaktor (4) oder den letzten Teil (z.B. die letzten 20 % der Gesamtverweildauer) der Verweildauer im Wesentlichen neutralen PH-Wert annähert. Eine Absenkung des PH-Werts im Hydrolysereaktor (4) kann durch im Hydrolyseprozess frei werdende organische Säuren realisiert sein. Vorzugsweise verbleibt der Gärrest 14 Stunden in den Hydrolysereaktor (4). Bei anderen Fahrweisen der Anlage kann die chemisch-thermische Hydrolyse zwischen 2 und 14 Stunden durchgeführt werden.

Während der chemischen Hydrolyse entstehen in dem Hydrolysereaktor (4) Hydrolysegase, die in den Gaswäscher (6) ausgeleitet werden. Durch die Gasausleitung nimmt der Stickstoff- und/oder Schwefelstoffanteil im hydrolysierten Gärrest ab, was sich positiv auf das aus dem hydrolysierten Gärrest zu gewinnende Gas und die Gasproduktion bei der Nachvergärung auswirkt. In dem Gaswäscher (6) wird das in den Hydrolysegasen enthaltene Ammoniak mit Schwefelsäure, die von der Schwefelsäurequelle (23) dem Gaswäscher (6) zugeführt wird, angereichert, um Ammoniumsulfat zu gewinnen.

Nachdem der Gärrest chemisch hydrolysiert wurde, wird dieser aus dem Hydrolysereaktor (4) zu dem Wärmetauscher (2) geleitet. Hierbei wird beispielsweise mit geeigneter Sensorik überprüft, welchen PH-Wert der Gärrest aufweist. Weicht der PH-Wert deutlich von einem gewünschten im Wesentlichen neutralen Niveau ab, wird mittels des Neutralisationsdosierers (20) eine Chemikalie dem Gärrest zugesetzt um den PH-Wert neutral einzustellen. Der Neutralisationsdosierer (20) und die von dem Neutralisationsdosierer an diese abgegebene Chemikalie sind auf die Dosiereinrichtung (8) und die von der Dosiereinrichtung (8) abgegebene Chemikalie in der Art eines chemischen Gegensatzpaars, wie Säure und Base, abgestimmt. Beispielsweise können auch die Regelalgorithmen des Neutralisationsdosierers (20) und der Dosiereinrichtung (8) miteinander kommunizierend ausgelegt sein, um den Chemikalienverbrauch zu minimieren.

In dem Wärmetauscher (2) wird der hydrolysierte Gärrest unter Verwendung des zweiten Betriebsmodus des Wärmetauschers (2) von der Hydrolysetemperatur, die bei etwa 80°C liegt, auf eine Nachvergärungstemperatur von etwa 55°C abgekühlt. Dadurch werden optimale mikrobielle Bedingungen für Nachvergärung geschaffen.

Anschließend wird der hydrolysierte Gärrest dem zweiten Fermentationsbehälter (5) zur Nachvergärung zugeführt. Der Gärrest kann im Fermentationsbehälter (5) mit über die Zufiihrleitung (19) einbringbarer frischer Biomasse angereichert werden. Der Fermentationsbehälter (5) ist wie der Fermentationsbehälter (1) und der Hydrolysereaktor (4) mit einer Heizeinrichtung und gegebenenfalls mit einer Temperaturregelung versehen, so dass die Nachvergärungstemperatur auf einem vorbestimmten Wert einstellbar ist. Vorzugsweise wird die Nachvergärungstemperatur auf zwischen 40°C und 55°C eingestellt. Der Gärrest und ggf. die zugeführte frische Biomasse verbleiben zwischen etwa 10 und etwa 40 Tage, vorzugsweise zwischen 20 und 30 Tage in dem Fermentationsbehälter (5). Beim Nachvergärvorgang wird aus dem hydrolysierten Gärrest durch Mikroorganismen Biogas produziert. Aufgrund der vorangehenden chemischen Hydrolyse liegt der Gärrest nun in einer für die Mikroorganismen leicht abbaubaren Form vor, so dass die Produktionsausbeute deutlich erhöht ist. Im Allgemeinen läuft der Nachvergärvorgang in gleicher Weise wie der Vorvergärprozess im Fermentationsbehälter (1) ab, jedoch mit dem Unterschied, dass jetzt ein größerer Anteil des Anlagensubstrats leicht mikrobiell verstoffwechselbar ist.

Anschließend an die Nachvergärung wird der Gärrest aus dem zweiten Fermentationsbehälter (5) in die Separiervorrichtung (9) geleitet, wo dieser in Flüssig- und Festbestandteile getrennt wird. Die Festbestandteile werden in das Feststofflager (7) ausgeleitet und die Flüssiganteile als Rezirkulat dem Fermentationsbehälter (1) zugeleitet. Teile des Gärrests können auch direkt aus dem zweiten Fermentationsbehälter (5) über eine Ablassleitung (15) in ein Endlager (10) ausgeleitet werden.

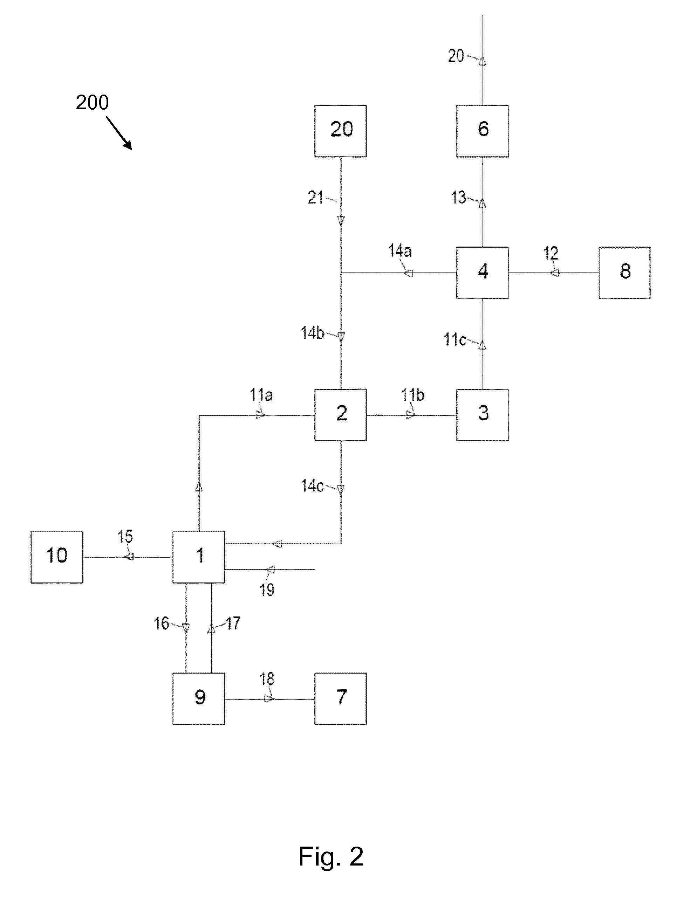

Eine alternative Ausführung einer erfindungsgemäßen Biogasanlage 200 ist in Fig. 2 dargestellt, in der Anlagenbestandteile der Biogasanlage 200, die entsprechend der Ausführung gemäß Fig. 1 ausgebildet sind, die gleichen Bezugsziffern aufweisen. Bei der Biogasanlage 200 ist nur ein einziger Fermentationsbehälter (1) vorgesehen und der zweite Fermentationsbehälter (5) entfällt. Der Aufbau und der Anlagenbetrieb unterscheiden sich von dem vorbeschriebenen Ausführungsbeispiel der Fig. 1 lediglich dadurch, dass der hydrolysierte Gärrest, der im Wärmetauscher (2) abgekühlt wird, zurück in den Fermentationsbehälter (1) geleitet wird. Der Nachvergärprozess läuft dann im Fermentationsbehälter (1) auf die gleiche Weise ab, wie dies zuvor für den Fermentationsbehälter (5) beschriebenen wurde. Gleiches gilt auch für das Abziehen des erzeugten Biogases. Die Separiervorrichtung (9) empfängt bei dem alternativen Ausführungsbeispiel den Gärrest des Fermentationsbehälters (1) über eine Zuleitung, die den Fermentationsbehälter (1) mit der Separiervorrichtung (9) verbindet.

Die in der vorstehenden Beschreibung, der Figur und den Ansprüche offenbarten Merkmale können sowohl einzeln als auch in beliebiger Kombination für die Realisierung der Erfindung in den verschiedenen Ausgestaltungen von Bedeutung sein.

### Bezugszeichenliste

- 1, 5: Fermentationsbehälter
- 2, 3: Wärmetauscher
- 4: Hydrolysereaktor
- 6: Gaswäscher
- 7: Feststofflager
- 8: Dosiereinrichtung
- 9: Separiervorrichtung
- 11a, 11b, 11c, 12, 14c, 24, 13, 21: Leitungen
- 14a, 14b, 15, 16: Ablassleitung
- 10: Endlager
- 17: Rezirkulatleitung
- 18: Feststoffleitung
- 19: Zuführleitung
- 20: Neutralisationsdosierer
- 22: Wertstoffauslass
- 23: Schwefelsäurequelle
- 100, 200: Biogasanlage

## Patentansprüche

1. Verfahren zum Erzeugen von Biogas aus organischer Biomasse, wie Energiepflanzen, Gülle, Festmist oder Bioabfällen, in einer Biogasanlage mit wenigstens einem Fermentationsbehälter (1, 5) und einem Hydrolysereaktor (4), bei dem:
- Biomasse unter Ausbildung eines Gärrests in dem wenigstens einen Fermentationsbehälter (1) vorvergoren wird;
- im Anschluss an das Vorvergären zumindest ein Anteil des Gärrests in dem Hydrolysereaktor (4) hydrolysiert wird; und
- der hydrolysierte Gärrest nachvergoren wird;
**dadurch gekennzeichnet, dass** der vorvergorene Gärrest chemisch hydrolysiert wird.

2. Verfahren nach Anspruch 1, **dadurch gekennzeichnet, dass** für die chemische Hydrolyse dem Gärsubstrat eine Chemikalie zum Bilden einer Gärrest-ChemikalienMischung zugegeben wird, wobei insbesondere die Chemikalie eine Säure oder Base enthält, wobei insbesondere die Chemikalie einen PH-Wert von wenigstens 12, vorzugsweise wenigstens etwa 13 aufweist oder einen PH-Wert von höchstens 3, vorzugsweise höchstens etwa 2 aufweist.

3. Verfahren nach einem der vorstehenden Ansprüche, **dadurch gekennzeichnet, dass** vor und/oder während der chemischen Hydrolyse der PH-Wert einer Gärrest-Chemikalien-Mischung auf zwischen 9 und 13, vorzugsweise auf zwischen etwa 10 und etwa 12 eingestellt wird.

4. Verfahren nach einem der vorstehenden Ansprüche, **dadurch gekennzeichnet, dass** der vorvergorene Gärrest aerob, vorzugsweise schwach aerob, hydrolysiert wird.

5. Verfahren nach einem der vorstehenden Ansprüche, **dadurch gekennzeichnet, dass** der vorvergorene Gärrest auf eine vorbestimmte Hydrolysetemperatur erwärmt wird, bevor und/oder während er hydrolysiert wird, wobei insbesondere die Hydrolysetemperatur zwischen 20°C und 100°C, insbesondere zwischen etwa 60°C und 90°C, vorzugsweise zwischen etwa 75°C und 85°C liegt.

6. Verfahren nach einem der vorstehenden Ansprüche, **dadurch gekennzeichnet, dass** der hydrolysierte Gärrest vor dem Nachvergären von einer Hydrolysetemperatur auf eine vorbestimmte Nachvergärungstemperatur abgekühlt wird, wobei insbesondere die Nachvergärungstemperatur zwischen etwa 20°C und etwa 70°C, vorzugsweise zwischen etwa 50°C und 60°C, liegt.

7. Verfahren nach einem der vorstehenden Ansprüche, **dadurch gekennzeichnet, dass** bei der Hydrolyse im Hydrolysereaktor (4) entstehende Gase aus dem Hydrolysereaktor entfernt werden, wobei insbesondere die entfernten Gase unter Verwendung eines Gaswäschers (6) mit Schwefelsäure angereichert werden.

8. Verfahren nach einem der vorstehenden Ansprüche, **dadurch gekennzeichnet, dass** der hydrolysierte Gärrest vor dem Nachvergären mit einer den PH-Wert verändernden Chemikalie behandelt wird, wobei insbesondere der PH-Wert des Gärrests im Wesentlichen neutral eingestellt wird.

9. Verfahren nach einem der vorstehenden Ansprüche, **dadurch gekennzeichnet, dass** der hydrolysierte Gärrest in demselben wenigstens einen Fermentationsbehälter (1) nachvergoren wird, in dem die Biomasse vorvergoren worden ist.

10. Biogasanlage (100) zur Erzeugung von Biogas aus organischer Biomasse, wie Energiepflanzen, Gülle, Festmist oder Bioabfällen, umfassend:
- einen Fermentationsbehälter (1), in den zu vergärende Biomasse oder ein zu vergärender Gärrest einbringbar ist und aus dem Biogas sowie ein vergorener Gärrest abziehbar sind; und
- einen Hydrolysereaktor (4), der mit dem Fermentationsbehälter (1) verbindbar ist, um den vergorenen Gärrest aus dem Fermentationsbehälter (1) aufzunehmen;
**gekennzeichnet durch** wenigstens eine Dosiereinrichtung (8), die mit einem Chemikalienspeicher und dem Hydrolysereaktor (4) verbunden und dazu ausgelegt ist, dem Hydrolysereaktor (4) eine definierte Menge einer Chemikalie zuzuführen.

11. Biogasanlage (100) nach Anspruch 10, **dadurch gekennzeichnet, dass** die Biogasanlage (100) wenigstens einen Wärmetauscher (2, 3) aufweist, der mit dem Fermentationsbehälter (1) und dem Hydrolysereaktor (4) verbindbar und dazu ausgelegt ist, einen aus dem Fermentationsbehälter (1) abegezogenen Gärrest zu erwärmen und/oder einen hydrolysierten Gärrest aus dem Hydrolysereaktor (4) abzukühlen.

12. Biogasanlage (100) nach einem der Ansprüche 10 oder 11, **dadurch gekennzeichnet, dass** der Hydrolysereaktor (4) chemikalienbeständig, insbesondere säuren- und/oder basenbeständig, ausgelegt ist, und/oder dass der Hydrolysereaktor (4) eine Belüftungsvorrichtung zum Zuführen von Sauerstoff in den Hydrolysereaktor (4) aufweist, die insbesondere eine Luftdrosseleinrichtung zum Einstellen der zugeführten Sauerstoffmenge umfasst.

13. Biogasanlage (100) nach einem der Ansprüche 10 bis 12, **dadurch gekennzeichnet, dass** die Biogasanlage einen Gaswäscher (6) mit einem Schwefelsäurespeicher aufweist, wobei der Gaswäscher (6) mit Hydrolysereaktor (4) verbunden und dazu ausgelegt ist, aus dem Hydrolysereaktor (4) ausgeleitetes Gas mit Schwefelsäure anzureichern.

14. Biogasanlage (100) nach einem der Ansprüche 10 bis 13, **dadurch gekennzeichnet, dass** sie einen zweiten Fermentationsbehälter (5) aufweist, der dem Hydrolysereaktor (4) nachgeschaltet ist.

15. Biogasanlage (100) nach einem der Ansprüche 10 bis 13, **dadurch gekennzeichnet, dass** sie nur einen Fermentationsbehälter (1) umfasst, der dem Hydrolysereaktor (4) sowohl vorgeschaltet als auch nachgeschaltet ist.
